# EUROPEAN PATENT SPECIFICATION

(11) **EP 2 889 267 B1**
(45) Date of publication and mention of the grant of the patent: **17.01.2018**
(21) Application number: 13831736.7
(22) Date of filing: 09.08.2013
(51) Int. Cl.: A61L 27/00, A61N 1/04, C08K 9/04, C08L 101/14

(54) **CARBON NANOMATERIAL, COMPOSITION, CONDUCTIVE MATERIAL, AND MANUFACTURING METHOD THEREFOR**
KOHLENSTOFFNANOMATERIAL, ZUSAMMENSETZUNG, LEITFÄHIGES MATERIAL UND HERSTELLUNGSVERFAHREN DAFÜR
NANOMATIÈRE DE CARBONE, COMPOSITION, MATIÈRE CONDUCTRICE ET SON PROCÉDÉ DE FABRICATION

(30) Priority: 23.08.2012 JP 2012184492
(43) Date of publication of application: 01.07.2015
(73) Proprietor: Japan Science and Technology Agency, Kawaguchi-shi Saitama 332-0012 (JP)
(72) Inventor: SEKITANI, Tsuyoshi, Tokyo 113-8654 (JP); SOMEYA, Takao, Tokyo 113-8654 (JP)
(74) Representative: Cabinet Plasseraud
(86) International application number: PCT/JP2013/071689
(87) International publication number: WO 2014/030556

(56) References cited:
- WO-A1-2009/101985
- WO-A1-2009/102077
- WO-A1-2011/055776
- JP-A- H09 158 054
- JP-A- 2001 164 419
- JP-A- 2004 523 665
- JP-A- 2005 176 428
- US-A1- 2006 257 645
- H. ZHANG ET AL: "Regenerated-Cellulose/Multiwalled- Carbon-Nanotube Composite Fibers with Enhanced Mechanical Properties Prepared with the Ionic Liquid 1-Allyl-3-methylimidazolium Chloride", ADVANCED MATERIALS, vol. 19, no. 5, 5 March 2007 (2007-03-05), pages 698-704, XP055079939, ISSN: 0935-9648, DOI: 10.1002/adma.200600442

## Description

### BACKGROUND OF THE INVENTION

### Field of the Invention

The present invention relates to a carbon nanomaterial, a composition, a conductive material, and a method of manufacturing the same.

### Description of Related Art

Recently, flexible electronics formed of organic semiconductors have attracted attention due to flexibility of a material thereof as means for directly obtaining biological information from cells or tissues by mounting them on the surface of the human body or in the body.

In the related art, when the electronics are used in the body, metal such as Pt or Au is used for electrical connection with tissues or cells in the body (NPLS 1 and 2).

However, when the metal such as Pt or Au comes into direct contact with tissues or cells in the body, an inflammatory reaction occurs between an electrode and tissues due to an antibody reaction of biological cells. Therefore, there is a problem in that biological information cannot be observed for a long period of time.

In addition, biological cells have undulations such as wrinkles on the surfaces thereof, and an electrode formed of metal is generally hard. Therefore, there is a problem in the followability to the surface shape. Therefore, contact with the electrode is unstable, and there is a problem in that, for example, an electrical signal is unstable.

As an electrode material which comes into direct contact with biological cells as described above, the development of a material having biocompatibility and superior followability to the surface shape, which can make the material sufficiently cover the surfaces of the biological cells, has been desired.

Then, a nanomaterial such as carbon nanotubes (CNT) is expected as a flexible conductive material.

PTL 1 discloses a conductive material for an actuator element having superior flexibility and an electrode layer for an actuator element, in which the conductive material consists of a gel formed of carbon nanotubes and an ionic liquid, and the electrode layer consists of a gel composition formed of carbon nanotubes, an ionic liquid, and a polymer. By performing a pulverization treatment under a shearing force during the formation of the gel or the gel composition, the degree of entanglement among the individual carbon nanotubes is decreased. As a result, it is thought that the gel or the gel composition is formed because molecules in ionic liquid, bonded to the surfaces of the less entangled carbon nanotubes through the "cation-π" interaction, serve to combine the bundles of carbon nanotubes with one another through ionic bonding (PTL 2). The "cation-π" interaction has a force comparable to the force of hydrogen bonding (approximately 10 times the Van der Waals force).
Document D1 (US 2006/257645) discloses an electroconductive film comprising a polymer gel containing carbon nanotubes with an aspect ratio of at least 10³, an ionic liquid, and a polymer.
Document D2 (H. Zhang et al.) discloses the surface of a cellulose fiber is coated with a carbon nanotube network (CNT) formed with an ionic liquid interposed therebetween. In other words, Document D2 discloses a polymer fiber is coated with a carbon nanotube network and an ionic liquid.
Document D3 (WO 2009/101985 A1) discloses a cellulose fiber is covered with a carbon nanotube.

### Citation List

### Patent Literature

[PTL 1] Japanese Patent No. 4038685
[PTL 2] Japanese Patent No. 3676337

### Non-Patent Literature

[NPL 1] Jonathan Vienti et-. al., Nature Neuroscience, 2011, Vol. 14, No. 12, 1599-1607
[NPL 2] Quan Qing et. al., PNAS, 2010, Vol. 107, No. 5, 1882-1887

### SUMMARY OF THE INVENTION

However, regarding the effects of a nanomaterial such as carbon nanotubes (CNT) on the living body have not been sufficiently studied, and the research on the use of the nanomaterial as a material of a device which is mounted on the surface of the human body or in the body has not progressed. In practice, it is not assumed that the materials disclosed in PTLS 1 and 2 are used as a material of a device which is mounted on the surface of the human body or in the body. Therefore, PTLS 1 and 2 neither describe nor imply a configuration which is included in the material in a state that the carbon nanotubes have no effect on the living body.

PTL 2 describes a configuration in which the ionic liquid molecules are bonded to the surfaces of the carbon nanotubes. However, PTL 2 neither describes nor implies a configuration in which the surfaces of the carbon nanotubes are covered with the ionic liquid molecules, and a layer of molecules constituting the ionic liquid is covered with a polymer.

In addition, PTL 1 discloses the electrode layer which consists of a gel composition formed of carbon nanotubes, an ionic liquid, and a polymer. However, the polymer is added to maintain the mechanical strength (for example, paragraph [0026]). Accordingly, PTL 1 merely discloses the electrode layer consisting of the gel composition which is obtained by using a method (for example, Example 1) including: heating and mixing carbon nanotubes, a gel of an ionic liquid, and a polymer with each other to form a gel composition; and forming an electrode layer by using the gel composition. In addition, PTL 1 neither describes nor implies a rinsing step of forming a single layer by using molecules constituting ionic liquid which cover carbon nanotubes, a rinsing step of removing molecules constituting ionic liquid which are not bonded to carbon nanotubes, or a crosslinking step of crosslinking a polymer.

The present invention has been made in consideration of the above-described circumstances, and an object thereof is to provide a carbon nanomaterial, a composition, a conductive material, and a method of manufacturing the same, in which the carbon nanomaterial, the composition, and the conductive material have biocompatibility, can be used in the living body for a long period of time, have superior followability to the shape of wrinkles of an organ or the like, and can form a far superior interface with an organ or the like.

The present inventors focused on the fact that molecules constituting ionic liquid are strongly bonded to surfaces of carbon nanotubes and, as a result of intensive study, conceived a revolutionary idea that the carbon nanotubes covered with the molecules constituting ionic liquid are further covered with a polymer. According to this material, the carbon nanotubes are doubly covered with the ionic liquid and the polymer and are not exposed from the surface of the material. As a result, even when the material is embedded into the living body, the contact of the carbon nanotubes with biological cells is avoidable. Regarding the material which was searched and discovered based on the idea, the present inventors performed "cytotoxicity test by using colony formation" according to International Standard ISO 10993-6 relating to biocompatibility and confirmed that the material did not have cytotoxicity. Further, the present inventors performed a "rabbit implantation test" according to the standard and confirmed that the degree of rejection of the living body was smaller than the case using an Au electrode of the related art, thereby completing the present invention.

In order to achieve the above-described object, the present invention adopt the following means.

According to an aspect of the present invention, a carbon nanomaterial is provided that is doubly covered with molecules constituting a hydrophilic ionic liquid and a water-soluble polymer.

Effects of the ionic liquid on the living body have not been sufficiently studied. However, the carbon nanomaterial according to the aspect of the present invention adopts the double-cover configuration of covering the molecules constituting the ionic liquid, which are bonded to the carbon nanomaterial, with the water-soluble polymer. Therefore, even when the carbon nanomaterial having this configuration is in contact with the living body, the contact of the molecules constituting the ionic liquid with the living body is avoidable. In addition, the main body of the carbon nanomaterial (carbon nanomaterial before being covered with the molecules constituting the ionic liquid and the water-soluble polymer) is doubly covered with the molecules constituting the ionic liquid and the water-soluble polymer. Therefore, even when the carbon nanomaterial having this configuration (doubly-covered carbon nanomaterial) is in contact with the living body, the contact of the main body of the carbon nanomaterial with the living body is avoidable.

According to another aspect of the present invention, a composition is provided including a carbon nanomaterial that is covered with molecules constituting a hydrophilic ionic liquid, in which the carbon nanomaterial is dispersed in a water-soluble polymer medium, and the carbon nanomaterial is doubly covered with the molecules constituting the ionic liquid and a water-soluble polymer.

Effects of the ionic liquid on the living body have not been sufficiently studied. However, the composition according to the aspect of the present invention adopts the double-cover configuration of covering the molecules constituting the ionic liquid, which are bonded to the carbon nanomaterial, with the water-soluble polymer. Therefore, even when the composition having this configuration is in contact with the living body, the contact of the molecules constituting the ionic liquid with the living body is avoidable. In addition, the main body of the carbon nanomaterial (carbon nanomaterial before being covered with the molecules constituting the ionic liquid and the water-soluble polymer) is doubly covered with the molecules constituting the ionic liquid and the water-soluble polymer. Therefore, even when the composition having this configuration is in contact with the living body, the contact of the main body of the carbon nanomaterial with the living body is avoidable.

This composition may be gel (not having fluidity) or liquid (having fluidity). In addition, a part of the composition may be gel and another part of the composition may be liquid.

The carbon nanomaterial dispersed in the water-soluble polymer medium may be carbon nanotubes.

In addition, "the water-soluble polymer medium" may be cured by applying energy (for example, heat, light, or electron beams).

In this specification, "being doubly coated" represents being covered with a layer of the molecules constituting the ionic liquid and a layer of the water-soluble polymer. In the composition according to the present invention, during the covering, the water-soluble polymer is in the form of a layer so as to cover the layer of the molecules constituting the ionic liquid in a state that the carbon nanomaterial covered with the layer of the molecules constituting the ionic liquid is mixed with the water-soluble polymer and water to disperse the water-soluble polymer in water, that is, in a state that particles of the water-soluble polymer having a small size are dispersed in water. On the other hand, "the electrode layer which consists of a gel composition formed of carbon nanotubes, an ionic liquid, and a polymer" described in PTL 1 is formed by heating and mixing carbon nanotubes, a gel of an ionic liquid, and a polymer with each other (for example, Example 1). Therefore, even when the ionic liquid molecules are covered with the carbon nanotubes in the form of a layer, the polymer does not cover the carbon nanotubes in the form of a layer (through the molecules constituting the ionic liquid).

In addition, in the composition according to the present invention, the carbon nanomaterial is covered with the molecules constituting the ionic liquid and the water-soluble polymer in the form of a layer. Therefore, the carbon nanomaterial can be covered with the layer of the molecules constituting the ionic liquid having a substantially uniform thickness and the layer of the water-soluble polymer having a substantially uniform thickness. That is, as compared to the gel composition described in PTL 1, the carbon nanomaterial represented by carbon nanotubes can be uniformly coated at a molecular level. In addition, the ionic liquid molecules are securely bonded to the carbon nanomaterial and thus can be coated without a pinhole.

The layer of the molecules constituting the ionic liquid having "a substantially uniform thickness" represents that a monomolecular layer accounts for 70% or higher, preferably, 90% or higher in the layer of the molecules constituting the ionic liquid.

In addition, the layer of the water-soluble polymer having "a substantially uniform thickness" represents that a variation in the thickness of the 70% or higher, preferably, 90% or higher of the layer of the water-soluble polymer is 20 nm or less, preferably 10 nm or less, and more preferably 5 nm or less.

By adjusting the amount of the hydrophilic ionic liquid so as to cover the entire carbon nanomaterial, a configuration can be adopted in which the molecules constituting the ionic liquid completely cover the carbon nanomaterial, and the water-soluble polymer covers the ionic liquid molecules.

The water-soluble polymer which covers the carbon nanomaterial with the molecules constituting the ionic liquid interposed therebetween; and the water-soluble polymer (medium) in which the covered carbon nanomaterial is dispersed may be the same type or different types. The composition having this composition can be obtained, for example, by using a method including: mixing the carbon nanomaterial covered with the molecules constituting the ionic liquid, the water-soluble polymer, and water with each other to cover the carbon nanomaterial with the water-soluble polymer with the molecules constituting the ionic liquid interposed therebetween; and mixing the carbon nanomaterial which is covered with the water-soluble polymer with the molecules constituting the ionic liquid interposed therebetween, another water-soluble polymer having the same type as or a different type from that of the above water-soluble polymer, and water with each other.

In the composition according to the aspect of the present invention, the carbon nanomaterial may be covered with a monomolecular film of the molecules constituting the ionic liquid.

The molecules constituting the ionic liquid are bonded to the surface of the carbon nanomaterial so as to cover the surface of the carbon nanomaterial, and the molecules constituting the ionic liquid molecules which are not bonded to the surface of the carbon nanomaterial are removed by rinsing. As a result, the carbon nanomaterial is covered with the monomolecular film of the molecules constituting the ionic liquid.

According to still another aspect of the invention, a biocompatible composition is provided including the composition according to any one of the above-described aspects.

In this specification, "biocompatibility" represents that a substance has no cytotoxicity and smaller degree of rejection of the living body than the case using an Au electrode. For example, "biocompatibility" represents that a substance has no cytotoxicity in a "cytotoxicity test by using colony formation" according to International Standard ISO 10993-6 relating to biocompatibility and has smaller degree of rejection of the living body than the case using an Au electrode in a "rabbit implantation test" according to the standard.

As described above, effects of the ionic liquid on the living body have not been sufficiently studied. However, the biocompatible composition according to the aspect of the present invention adopts the double-cover configuration of covering the molecules constituting the ionic liquid, which are bonded to the carbon nanomaterial, with the water-soluble polymer. Therefore, even when the carbon nanomaterial having this configuration is in contact with the living body, the contact of the molecules constituting the ionic liquid with the living body is avoidable. In addition, the main body of the carbon nanomaterial (carbon nanomaterial before being covered with the molecules constituting the ionic liquid and the water-soluble polymer) is doubly covered with the molecules constituting the ionic liquid and the water-soluble polymer. Therefore, even when the biocompatible composition having this configuration is in contact with the living body, the contact of the main body of the carbon nanomaterial with the living body is avoidable.

According to still another aspect of the present invention, a conductive material is provided including a carbon nanomaterial that is doubly covered with molecules constituting a hydrophilic ionic liquid and a water-soluble polymer, in which the carbon nanomaterial is dispersed in a water-soluble polymer medium, and the water-soluble polymer is crosslinked.

By adjusting the amount of the hydrophilic ionic liquid so as to cover the entire carbon nanomaterial, a configuration can be obtained in which the ionic liquid molecules cover the carbon nanomaterial, and the water-soluble polymer covers the ionic liquid molecules.

The carbon nanomaterial dispersed in the water-soluble polymer medium may be carbon nanotubes.

According to still another aspect of the present invention, a biocompatible conductive material is provided including a carbon nanomaterial that is doubly covered with molecules constituting a hydrophilic ionic liquid and a water-soluble polymer, in which the carbon nanomaterial is dispersed in a water-soluble polymer medium, and the water-soluble polymer is crosslinked.

As described above, effects of the ionic liquid on the living body have not been sufficiently studied. However, the biocompatible conductive material according to the aspect of the present invention adopts the double-cover configuration of covering the molecules constituting the ionic liquid, which are bonded to the carbon nanomaterial, with the water-soluble polymer. Therefore, even when the carbon nanomaterial having this configuration is in contact with the living body, the contact of the molecules constituting the ionic liquid with the living body is avoidable. In addition, the main body of the carbon nanomaterial (carbon nanomaterial before being covered with the molecules constituting the ionic liquid and the water-soluble polymer) is doubly covered with the molecules constituting the ionic liquid and the water-soluble polymer. Therefore, even when the biocompatible conductive material having this configuration is in contact with the living body, the contact of the main body of the carbon nanomaterial with the living body is avoidable.

According to still another aspect of the present invention, a method of manufacturing a conductive material is provided, the method including: a first step of mixing a hydrophilic ionic liquid, a carbon nanomaterial, and water with each other to prepare a first dispersion system in which the carbon nanomaterial covered with the molecules constituting the ionic liquid is dispersed; and a second step of mixing the first dispersion system, a water-soluble polymer, and water with each other to prepare a second dispersion system in which the carbon nanomaterial covered with the molecules constituting the ionic liquid and the water-soluble polymer are dispersed.

In the second dispersion system, by using plural types of water-soluble polymers, the water-soluble polymer which covers the carbon nanomaterial with the molecules constituting the ionic liquid interposed therebetween; and the water-soluble polymer (medium) in which the covered carbon nanomaterial is dispersed can be made to be different types. Alternatively, plural types of water-soluble polymers which cover the carbon nanomaterial with the ionic liquid molecules interposed therebetween may be used. As a result, the hardness of the material can be adjusted, and the conductivity, optical characteristics, and the like thereof can be controlled.

In the method of manufacturing a conductive material according to the aspect of the present invention, in the first step, the carbon nanomaterial may be pulverized by applying a shearing force to the carbon nanomaterial.

The method of manufacturing a conductive material according to the aspect of the present invention may further include, after the second step, a step of preparing a crosslinked composition in the water-soluble polymer by crosslinking the water-soluble polymer and dispersing the carbon nanomaterial in the water-soluble polymer medium.

After the crosslinking step, the composition is mixed with another water-soluble polymer having a different type from that of the water-soluble polymer used in the second step, and the mixture is crosslinked. As a result, the hardness of the material can be adjusted, and the conductivity, optical characteristics, and the like thereof can be controlled.

The method of manufacturing a conductive material according to the aspect of the present invention may further include a rinsing step of removing the molecules constituting the ionic liquid which are not bonded to the carbon nanomaterial. This rinsing step can be performed by using, for example, saline solution, ethanol, or liquid which does not destroy gel.

As described above, effects of the ionic liquid on the living body have not been sufficiently studied. However, the method of manufacturing a conductive material according to the aspect of the present invention includes the rinsing step. As a result, a conductive material can be manufactured in which the molecules constituting the ionic liquid, which are bonded to the carbon nanomaterial, are covered with the water-soluble polymer. Therefore, in the obtained conductive material, the contact of the molecules constituting the ionic liquid with biological cells is avoided.

This rinsing step may be performed at any time, for example, after the first step, after the second step, or the after the step of preparing a composition.

By appropriately performing the rinsing step after the first step, a monolayer can be securely formed by using the molecules constituting the ionic liquid which covers the carbon nanomaterial. In addition, by appropriately performing the rinsing step, for example, after the second step or after crosslinking the water-soluble polymer, the molecules constituting the ionic liquid which are not bonded to the carbon nanomaterial can be removed. After the crosslinking of the water-soluble polymer, the water-soluble polymer is not dissolved in the liquid used in the rinsing step. Therefore, the molecules constituting the ionic liquid are easily removed.

The carbon nanomaterial, the composition, the biocompatible composition, the conductive material, the biocompatible conductive material, and the method of manufacturing a conductive material according to the above-described aspects of the present invention are not particularly limited to the use in the living body and can be used in various fields where the effects thereof can be exhibited.

The carbon nanomaterial, the composition, the biocompatible composition, the conductive material, or the biocompatible conductive material according to the present invention adopts a configuration in which the carbon nanomaterial included therein is doubly covered with the ionic liquid molecules and the water-soluble polymer. Therefore, even when they are used in the living body, the carbon nanomaterial does not substantially come into contact with cells in the living body (alternatively, the contact area with the cells is significantly decreased). In addition, due to high flexibility, the followability to the surface of an organ or the like in the living body is superior, and a far superior interface can be formed with an organ or the like. Further, high conductivity can be obtained, for example, by adjusting the content of the carbon nanomaterial. In addition, the conductivity between the carbon nanomaterials can be improved by forming a monomolecular film by using the molecules constituting the ionic liquid which cover the carbon nanomaterial. The water-soluble polymer which covers the carbon nanomaterial with the molecules constituting the ionic liquid interposed therebetween; and the water-soluble polymer (medium) in which the covered carbon nanomaterial is dispersed are made to be different types. As a result, only one of the water-soluble polymers can be cured.

In the related art, as in the case of a stimulating electrode in a device such as a pacemaker, an electrode which stimulates an organ or the like in the living body can be used in the living body for a long period of time. However, an electrode which reads a signal from an organ or the like in the living body cannot be used in the living body for a long period of time. This is because, when an electrode formed of a material in the related art is embedded into the living body, a foreign-body reaction (inflammatory reaction) occurs shortly after between the electrode and a tissue such as an organ, and it is difficult to detect an electrical signal. In the stimulating electrode, even when a foreign-body reaction occurs, an object of stimulating an organ or the like can be achieved. However, in the electrode which detects a signal from an organ or the like in the living body, it is difficult to achieve an object of detecting the signal.

On the other hand, the carbon nanomaterial, the composition, the biocompatible composition, the conductive material, or the biocompatible conductive material according to the present invention can be embedded into the living body for a long period of time. The composition, the biocompatible composition, the conductive material, or the biocompatible conductive material according to any one of the aspects of the present invention is the first material capable of being used in an electrode or the like which stably reads an electrical signal from an organ or the like in the living body for a long period of time.

In this way, the carbon nanomaterial, the composition, the biocompatible composition, the conductive material, or the biocompatible conductive material according to the present invention has a low antibody reaction even when being embedded into the living body for a long period of time and can be used as a material of a highly reliable electrode for the living body. In addition, the carbon nanomaterial, the composition, the biocompatible composition, the conductive material, or the biocompatible conductive material according to the present invention is extremely flexible and thus can cover the surface of a biological tissue without damaging the biological tissue. In addition, in regard to a cellular tissue having a size of several hundreds of micrometers, the carbon nanomaterial, the composition, the biocompatible composition, the conductive material, or the biocompatible conductive material according to the present invention can be used to manufacture a fine electrode which can be used in the cellular tissue because they can form photo-crosslinking.

In addition, an electrode which is manufactured by using the carbon nanomaterial, the composition, the biocompatible composition, the conductive material, or the biocompatible conductive material according to the present invention can come into close contact with an organ or the like in the living body to stably read a signal from the organ or the like in the living body for a long period of time. Therefore, the signal read from the organ or the like can be amplified beside the organ or the like by using an organic transistor (for example, NATURE MATERIALS, 9, 2010, 1015-1022). As a result, an extremely weak signal can be read with high accuracy. That is, although it is difficult to detect a weak signal from the living body without amplification, the weak signal from the living body can be detected by amplifying the weak signal beside an organ or the like in the living body by using a flexible amplifier which is manufactured from the organic transistor and an electrode which is manufactured from the carbon nanomaterial, the composition, the biocompatible composition, the conductive material, or the biocompatible conductive material according to the present invention.

In addition, when an electrical signal of an organ or the like is detected by capacitive coupling, the strength thereof is proportional to the surface area of the electrode. When an electrical signal is detected by capacitive coupling by using an electrode which is manufactured from the carbon nanomaterial, the composition, the biocompatible composition, the conductive material, or the biocompatible conductive material according to any one of the aspects of the present invention, this electrode has a large substantial contact area because it is far more flexible than a metal electrode of the related art and can be closely attached onto an organ or the like. Therefore, the detection sensitivity at a substantial capacity for obtaining an electrical signal is extremely higher than that of a metal electrode of the related art, and thus the size of the electrode can be further reduced. In addition, the carbon nanomaterial, the composition, the biocompatible composition, the conductive material, or the biocompatible conductive material according to any one of the aspects of the present invention includes a carbon nanomaterial having a high specific surface area. Even from this point of view, they have high signal detectability.

In the method of manufacturing a conductive material according to the aspect of the present invention, a conductive material having desired conductivity can be manufactured by adjusting the type and the content of the carbon nanomaterial. In addition, the carbon nanomaterial in which bundling or aggregation is suppressed is covered with the water-soluble polymer with the molecules constituting the ionic liquid interposed therebetween. As a result, as compared to a case where the carbon nanomaterial is covered with the water-soluble polymer without the molecules constituting the ionic liquid, a conductive material which is covered with the water-soluble polymer having a more uniform thickness can be manufactured. In addition, the layer of the molecules constituting the ionic liquid which cover the carbon nanomaterial can be made to be a monomolecular layer by selecting a combination of the carbon nanomaterial and the ionic liquid in which a bonding strength between the surface of the carbon nanomaterial and the molecules constituting the ionic liquid which is obtained through the "cation-π" interaction is higher than a bonding strength between the molecules constituting the ionic liquid. Further, by selecting the water-soluble polymer such that the monomolecular layer of the ionic liquid can be formed to be thin, the density of the carbon nanomaterial can be increased, and a conductive material having higher conductivity can be manufactured.

### BRIEF DESCRIPTION OF THE DRAWINGS

FIG. 1A, 1B, and 1C show a composition or a conductive material in the present invention, in which FIG 1A is an image showing a composition obtained by dispersing carbon nanotubes, covered with molecules constituting DEMEBF₄, in polyrotaxane; FIG 1B is an image of a sheet obtained by photo-crosslinking the composition shown in FIG 1A; and FIG 1C is an optical microscope image showing a state after photo-crosslinking the composition shown in FIG. 1A and patterning a fine structure having a line width of about 50 µm.

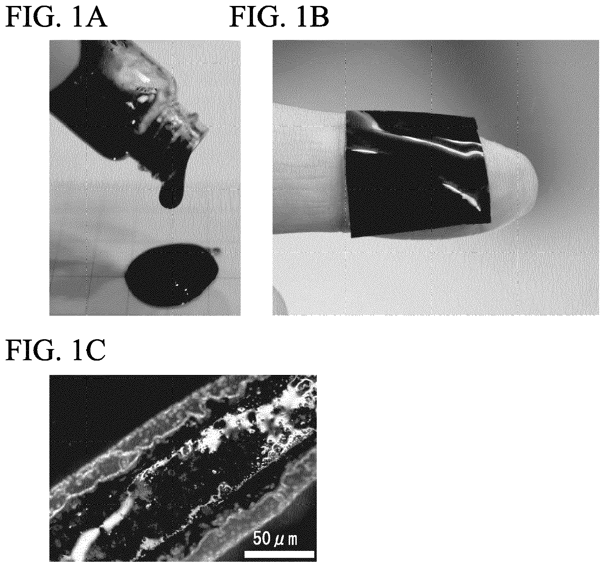

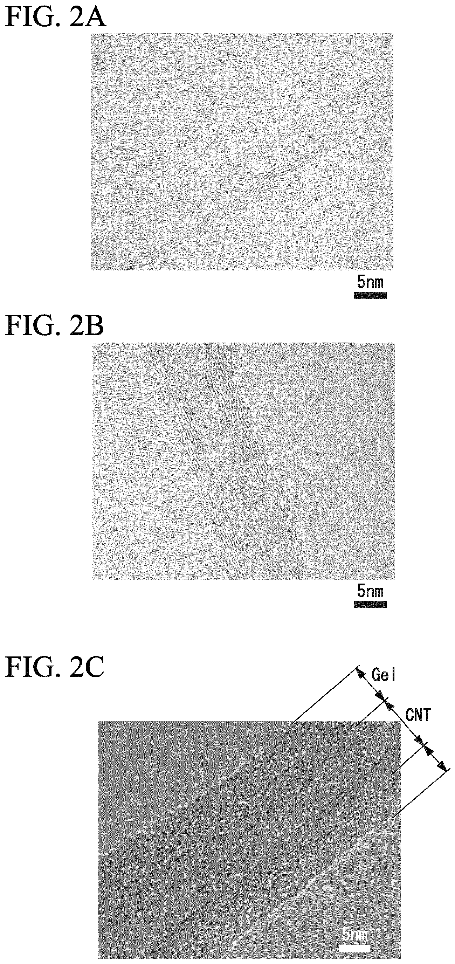

FIG. 2A, 1B, and 1C show high-resolution cross-sectional transmission electron microscope images (TEM images), in which FIG2A is a TEM image showing a carbon nanotube which can be used in the present invention; FIG2B is a TEM image showing a carbon nanotube covered with polyrotaxane, the carbon nanotube being obtained by mixing a carbon nanotube and polyrotaxane with each other in water without an ionic liquid and stirring the mixture while pulverizing the mixture with a jet mill; and FIG2C is a TEM image showing a carbon nanomaterial or a composition obtained under the same conditions as the production conditions of the composition shown in FIG. 1A.
FIG. 3 is a graph showing the surface resistance of a composition (or a conductive material) according to the present invention and the dependency thereof on the carbon nanotube content.
FIG. 4 is a graph showing the electrical capacitance of the present invention (or a conductive material) and the dependency thereof on the frequency.
FIG. 5 is a flowchart showing a method of manufacturing a conductive material according to the present invention.
FIG. 6 is a flowchart showing an application example of the method of manufacturing a conductive material according to the present invention.

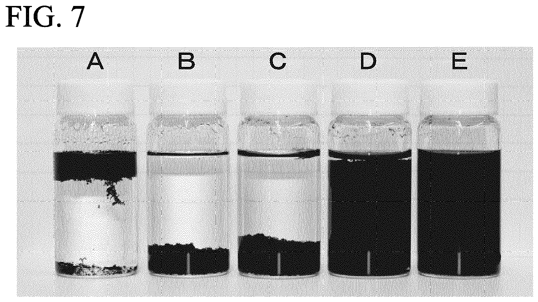

FIG. 7 shows images of the results of investigating the dispersibility of carbon nanotubes, in which (A) is an image showing a state after carbon nanotubes were put into deionized water and were stirred for 1 week; (B) is an image showing a state after carbon nanotubes and DEMEBF₄ were put into deionized water and were stirred for 1 week in the same manner; (C) is an image showing a state after carbon nanotubes were put into deionized water, were stirred for 1 week in the same manner, and were processed with a jet mill; (D) is an image showing a state after carbon nanotubes and 60 mg of DEMEBF₄ were put into deionized water, were stirred for 1 week in the same manner, and were processed with a jet mill; (E) is an image showing a state after carbon nanotubes, DEMEBF₄, and microfibrillated cellulose were put into deionized water and were stirred for 1 week in the same manner to obtain a paste, and then the paste was processed with a jet mill.

### DETAILED DESCRIPTION OF THE INVENTION

Hereinafter, embodiments of the present invention will be described with reference to the drawings.

According to an embodiment of the present invention, a carbon nanomaterial is provided that is doubly covered with molecules constituting a hydrophilic ionic liquid and a water-soluble polymer.

For example, this doubly covered carbon nanomaterial may be mixed into paper to prepare conductive paper. As a result, an organ or the like in the living body can come into contact with the conductive paper without directly contacting the carbon nanomaterial. Similarly, this doubly covered carbon nanomaterial may be mixed with a material other than paper to prepare a product. As a result, an organ or the like in the living body can come into contact with the product without directly contacting the carbon nanomaterial.

According to another embodiment of the present invention, a composition is provided including a carbon nanomaterial that is covered with molecules constituting a hydrophilic ionic liquid, in which the carbon nanomaterial is dispersed in a water-soluble polymer medium, and the carbon nanomaterial is doubly covered with the molecules constituting the ionic liquid and a water-soluble polymer.

It is preferable that the carbon nanomaterial be covered with a monomolecular film of the molecules constituting the ionic liquid.

According to still another embodiment of the present invention, a conductive material is provided including a carbon nanomaterial that is doubly covered with molecules constituting a hydrophilic ionic liquid and a water-soluble polymer, in which the carbon nanomaterial is dispersed in a water-soluble polymer medium, and the water-soluble polymer is crosslinked.

In this specification, the ionic liquid is also referred to as an ordinary temperature molten salt or simply as a molten salt and is defined as a salt which is in a molten state in a wide temperature range including ordinary temperature.

Among various hydrophilic ionic liquids which are known in the related art, a hydrophilic ionic liquid can be used, and examples thereof include N,N-diethyl-N-methyl-N-(2-methoxyethyl)ammonium tetrafluoroborate (DEMEBF₄).

In this specification, the carbon nanomaterial refers to a material in which a constituent element (for example, one CNT) having a nanometer-size structure is formed of carbon atoms, and the carbon atoms of the constituent element are bonded to each other with the Van der Waals force. Examples of the carbon nanomaterial includes carbon nanotubes, carbon nanofibers (carbon fibers having a diameter of 10 nm or less), carbon nanohorns, and fullerenes. When a fine carbon nanomaterial having a diameter of 10 nm or less is used, superior dispersibility is exhibited in water.

The same type of carbon nanomaterial may be used, or different types of carbon nanomaterials may be used.

Carbon nanotubes have a structure in which a single-layer or multi-layer graphene sheet with hexagonally arranged carbon atoms is rolled up in a cylindrical shape (referred to as single-wall nanotubes (SWNTs), double-wall nanotubes (DWNTs), or multi-wall nanotubes (MWNTs)). The carbon nanotubes which can be used as the carbon nanomaterial are not particularly limited and may be any one of SWNTs, DWNTs, and MWNTs. In addition, generally, carbon nanotubes can be manufactured by using, for example, a laser ablation method, arc discharge, a thermal CVD method, a plasma CVD method, a gas-phase method, or a combustion method. In addition, plural types of carbon nanotubes may be used.

The carbon nanotubes are likely to aggregate due to the Van der Waals force between the carbon nanotubes and are present in a state that plural carbon nanotubes form a bundle or an aggregate. However, the bundle or the aggregate can be pulverized (the degree of entanglement among the carbon nanotubes can be decreased) by applying a shearing force thereto in the presence of the ionic liquid. By sufficiently pulverizing the bundle or the aggregate, the the Van der Waals force, which causes the carbon nanotubes to aggregate, is weakened, the carbon nanotubes can be separated into individual carbon nanotubes, and the ionic liquid can be adsorbed onto the individual carbon nanotubes. As a result, a composition consisting of an ionic liquid and carbon nanotubes, which includes a single carbon nanotube covered with the molecules constituting the ionic liquid, can be obtained.

The means for applying a shearing force which is used in the pulverizing step is not particularly limited, and a wet pulverizer which can apply a shearing force, for example, a ball mill, a roller mill, or a vibrating mill can be used.

The carbon nanotubes and the ionic liquid are mixed with each other, and then the pulverizing step is performed. As a result, it is thought that the gel composition is formed because the molecules constituting the ionic liquid, which is bonded to the surfaces of the less entangled carbon nanotubes through the "cation-π" interaction, serve to combine the carbon nanotubes with one another through ionic bonding (PTL 2). As described below, by rinsing this gel composition by using, for example, saline solution or ethanol, a single layer of the molecules constituting the ionic liquid can be formed on the surfaces of the carbon nanotubes. Further by mixing water and the water-soluble polymer with the carbon nanotubes covered with the molecules constituting the ionic liquid, a composition can be manufactured in which the carbon nanotubes covered with the molecules constituting the ionic liquid are dispersed in a water-soluble polymer medium.

In this specification, the water-soluble polymer (medium) is not particularly limited as long as it is a polymer which can be dissolved or dispersed in water, and it is more preferable that the water-soluble polymer can be crosslinked in water. For example, the following examples can be used.

### 1. Synthetic Polymer

### (1) Ionic

Polyacrylic acid (anionic)
Polystyrene sulfonic acid (anionic)
Polyethyleneimine (cationic)
MPC polymer (amphoteric)

### (2) Nonionic

Polyvinylpyrrolidone (PVP)
Polyvinyl alcohol (saponified polyvinyl acetate)
Polyacrylamide (PAM)
Polyethylene oxide (PEO)

### 2. Natural Polymer (Mostly Polysaccharides)

Starch
Gelatin
Hyaluronic acid
Alginic acid
Dextran
Protein (for example, water-soluble collagen)

### 3. Semi-synthetic Polymer (For Example, Solubilized Cellulose)

Cellulose derivatives such as carboxymethyl cellulose (CMC), hydroxypropyl cellulose (HPC), or methyl cellulose (MC)
Water-soluble chitosan (which may also be classified into "2. Natural Polymer")

In addition, specific examples of the water-soluble polymer include polyrotaxane. Polyrotaxane is obtained by disposing a blocking group at both terminals of pseudo-polyrotaxane (both terminals of a linear molecule) so as to prevent a cyclic molecule from leaving, the pseudo-polyrotaxane having a structure in which the linear molecule (axis) is included in a cavity of the cyclic molecule (rotator) in a state of being skewered. For example, polyrotaxane containing α-cyclodextrin as the cyclic molecule and polyethylene glycol as the linear molecule can be used.

In addition, as the water-soluble polymer medium, a compound having a group, which is reactive with a crosslinking agent, is more preferably because it forms a firm film by crosslinking.

In order to form a fine-shaped pattern by using the composition or the conductive material according to the present invention, it is preferable that the water-soluble polymer be photo-crosslinkable polymer.

The layer of the molecules constituting the ionic liquid which cover the carbon nanomaterial may be a monomolecular layer. The molecules constituting the ionic liquid are bonded to the surface of the carbon nanomaterial through the "cation-π" interaction. Therefore, the layer of the molecules constituting the ionic liquid which cover the carbon nanomaterial can be made to be a monomolecular layer by selecting a combination of the carbon nanomaterial and the ionic liquid in which a bonding strength between the molecules constituting the ionic liquid is lower than a bonding strength which is obtained through the "cation-π" interaction.

For example, the layer of molecules of N,N-diethyl-N-methyl-N-(2-methoxyethyl)ammonium tetrafluoroborate (DEMEBF₄) which cover the carbon nanomaterial can be made to be a monomolecular layer by selecting carbon nanotubes as the carbon nanomaterial and selecting DEMEBF₄ as the ionic liquid. Further, for example, when polyrotaxane is selected as the water-soluble polymer, a thin layer of polyrotaxane having a thickness of about 5 nm can be formed on the monomolecular layer of DEMEBF₄. In a composition obtained as above, the dispersion concentration of the carbon nanotubes can be made to be high, and a material having high conductivity can be obtained. In a conductive member such as an electrode which is manufactured by using the conductive material, electrons migrate between the carbon nanotubes through the thin DEMEBF₄ molecular layer and the thin polyrotaxane layer.

In the composition or the conductive material according to the present invention, the molecules constituting the ionic liquid are strongly bonded to the surface of the carbon nanomaterial through the "cation-π" interaction. Therefore, the molecules constituting the ionic liquid which are bonded to the surface of the carbon nanomaterial are not released outside the water-soluble polymer medium. The molecules constituting the ionic liquid which are not bonded to the surface of the carbon nanomaterial are removed by rinsing by using, for example, saline solution or ethanol.

In the composition or the conductive material according to the present invention, the carbon nanomaterial included therein is doubly covered with the molecules constituting the ionic liquid molecules and the water-soluble polymer. Therefore, even when the composition or the conductive material according to the present invention is used in the living body, the carbon nanomaterial does not substantially come into contact with cells in the living body. In addition, due to high flexibility, the followability to the surface of an organ or the like in the living body is superior, and a far superior interface can be formed with an organ or the like. Further, high conductivity can be obtained.

According to still another embodiment of the present invention, a method of manufacturing a conductive material is provided, the method including: a first step of mixing a hydrophilic ionic liquid, a carbon nanomaterial, and water with each other to prepare a first dispersion system in which the carbon nanomaterial covered with the molecules constituting the ionic liquid is dispersed; and a second step of mixing the first dispersion system, a water-soluble polymer, and water with each other to prepare a second dispersion system in which the carbon nanomaterial covered with the molecules constituting the ionic liquid and the water-soluble polymer are dispersed.

In the first step, the carbon nanomaterial may be pulverized by applying a shearing force to the carbon nanomaterial.

As a result, the carbon nanomaterial can be covered with the hydrophilic ionic liquid in a state that a bundle or an aggregate of the carbon nanomaterial is further separated.

The method of manufacturing a conductive material may further include, after the second step, a step of preparing a composition by crosslinking the water-soluble polymer and dispersing the carbon nanomaterial in the water-soluble polymer medium. As a result, moldability and processability can be improved.

The method of manufacturing a conductive material may further include a rinsing step of removing the molecules constituting the ionic liquid which are not bonded to the carbon nanomaterial. As a result, moldability and processability can be improved.

This rinsing step can be performed by using, for example, saline solution, ethanol, or liquid which does not destroy gel. This rinsing step may be performed at any time.

The composition or the conductive material according to the present invention may further include other materials within a range not impairing the effects of the present invention. The method of manufacturing a conductive material according to the present invention may further include other steps within a range not impairing the effects of the present invention.

Hereinafter, the present invention will be described in detail based on examples. However, these examples are described to help easy understanding of the present invention, and the present invention is not limited to these examples. Examples

FIG. 1A is an image showing a composition before being cured with ultraviolet (UV) rays, the composition being obtained by dispersing carbon nanotubes, which is covered with molecules constituting N,N-diethyl-N-methyl-N-(2-methoxyethyl)ammonium tetrafluoroborate (DEMEBF₄), in polyrotaxane. It can be seen that the obtained composition was gel (in this specification, "gel" refers to a state in which there is no fluidity or substantially no fluidity relative to liquid having fluidity).

In order to prepare this composition, 30 mg of commercially available carbon nanotubes (MWNTs, length: 10 µm, diameter: 5 nm) and 60 mg of N,N-diethyl-N-methyl-N-(2-methoxyethyl)ammonium tetrafluoroborate (DEMEBF₄) as a hydrophilic ionic liquid were mixed with each other and were stirred in deionized water at 25°C for 1 week by using a magnetic stirrer at a rotating speed of 700 rpm or higher. The obtained suspension was processed with a high-pressure jet-milling homogenizer (60 MPa; Nano-jet Pal, JN10, Jokoh) to obtain a black material. A solution including the obtained CNT gel was rinsed with saline solution, and then 1 mg of a photo-crosslinking agent (Irgacure 2959, manufactured by Nagase & Co., Ltd.) and 1000 mg of polyrotaxane gel ("photo-crosslinkable gel", manufactured by Advanced Softmaterials Inc.) were mixed with the solution to prepare the above-described composition.

FIG. 1B is an image showing a sheet obtained by irradiating the composition shown in FIG. 1A with ultraviolet rays (wavelength: 365 nm) for 5 minutes to be cured.

The Young's modulus of the obtained sheet was lower than 10 kPa. The Young's modulus of silicon is about 100 GPa, and the Young's modulus of a plastic film of the related art is 1 GPa to 5 GPa. Therefore, it can be seen that the sheet was extremely flexible. In addition, the Young's modulus of a brain is 1 kPa to 2 kPa, and the Young's modulus of muscle cells of a heart is 100 kPa or less. Therefore, it can be seen that the composition or the conductive material according to the embodiment of the present invention has high flexibility equal to or higher than that of an organ. Therefore, the followability to the surface of an organ is high, and a far superior interface can be formed with an organ.

FIG. 1C is an optical microscope image showing a state after photo-crosslinking the composition by using a ultrafine digital UV exposure system ("digital exposure device" manufactured by PMT Corporation) and patterning a fine structure having a line width of about 50 µm. The composition or the conductive material according to the embodiment of the present invention is a material with which fine processing can be performed.

By changing the type of the photo-crosslinkable material, crosslinking can be performed at various wavelengths. Therefore, the means of crosslinking is not limited toUV.

FIG. 2 is high-resolution cross-sectional transmission electron microscope images (TEM images), in which FIG 2A is a TEM image showing a carbon nanotube (MWNT, length: 10 µm, diameter: 5 nm) which can be used in the present invention; FIG 2B is a TEM image showing a carbon nanotube covered with polyrotaxane, the carbon nanotube being obtained by mixing 30 mg of a carbon nanotube (MWNT, length: 10 µm, diameter: 5 nm) and 100 mg of polyrotaxane ("photo-crosslinkable gel", manufactured by Advanced Softmaterials Inc.) with each other in water without an ionic liquid and stirring the mixture while pulverizing the mixture with a jet mill; and FIG 2C is a TEM image showing a composition obtained under the same conditions as the production conditions of the composition shown in FIG. 1(a).

As a high-resolution cross-sectional transmission electron microscope, HF-2000 Cold-FE TEM (80 kV, manufactured by Hitachi High-Technologies Corporation) was used.

As shown in FIG. 2A, it can be seen that the used carbon nanotube consisted of three layers or four layers.

As shown in FIG. 2B, it can be seen that the single carbon nanotube was covered with polyrotaxane, but the thickness of the coating layer thereof was not uniform. On the other hand, as shown in FIG. 2C, it can be seen that the thickness of the polyrotaxane layer covering the single carbon nanotube was extremely uniform and was clearly different from that of FIG. 2B.

The difference in uniformity between the thicknesses of the coting layers shows that the carbon nanotube shown in FIG. 2C was obtained by covering the layer of the molecules of the hydrophilic ionic liquid DEMEBF₄, which had covered the carbon nanotube, with polyrotaxane, not by covering the carbon nanotube with polyrotaxane after removing the molecules of the hydrophilic ionic liquid DEMEBF₄, which had covered the carbon nanotube, from the carbon nanotube. If the carbon nanotube shown in FIG. 2C was obtained by covering the carbon nanotube with polyrotaxane after removing the molecules of the hydrophilic ionic liquid DEMEBF₄, which had covered the carbon nanotube, from the carbon nanotube, the thickness of the coating layer shown in FIG. 2C would be nonuniform as in the case of FIG. 2B. In addition, It is considered that, since the molecules of DEMEBF₄ were bonded to the carbon nanotube through cation-π interaction having a strong force comparable to the force of hydrogen bonding, the molecules of the hydrophilic ionic liquid DEMEBF₄ covering the carbon nanotube were not removed from the carbon nanotube.

As shown in FIG. 2, with the method of manufacturing a conductive material according to the present invention, a surface of a carbon nanotube can be uniformly covered with a biocompatible material with molecules constituting the ionic liquid interposed therebetween.

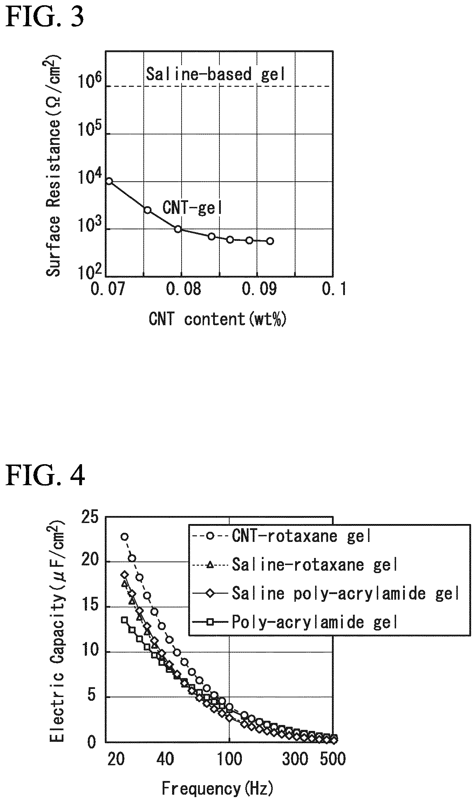

FIG. 3 is a graph showing the surface resistance of a composition (CNT-gel) according to the present invention and the carbon nanotube content dependency of the surface resistance. For comparison, the surface resistance of a gel (Saline-based gel) of the related art containing saline solution as a main component is also indicated by a dotted line.

The composition (CNT-gel) was obtained under the same conditions as the production conditions of the composition shown in FIG. 1A. The size of it was 1 cm x 1 cm, and the thickness of it was 1 mm.

The gel (Saline-based gel) containing saline solution as a main component was obtained by adding 1 mg of a photo-crosslinking agent to 300 mg of rotaxane gel, dissolving the mixture in 100 ml of saline solution, and then photocrosslinking the solution by using UV rays. The size of it was 1 cm × 1 cm, and the thickness of it was 1 mm.

As shown in FIG. 3, it can be seen that the surface resistance of the composition according to the embodiment of the present invention is lower by more than two or three digits than that of the gel of the related art containing saline solution as a main component.

FIG. 4 is a graph showing the electrical capacitance of a composition (CNT-rotaxane gel) according to the present invention and the frequency dependency of the electrical capacitance. For comparison, the electrical capacitances of a polyacrylamide gel (Poly-acrylamide gel), a saline solution-containing polyacrylamide gel (Saline poly-acrylamide gel), a saline solution-containing rotaxane gel (Saline-rotaxane gel) are also indicated.

The composition (CNT-rotaxane gel) was obtained under the same conditions as the production conditions of the composition shown in FIG. 1A. The size of it was 1 cm x 1 cm, and the thickness of it was 1 mm.

The polyacrylamide gel (Poly-acrylamide gel) was obtained by adding 1 mg of a photo-crosslinking agent to 300 mg of polyacrylamide, dissolving the mixture in 100 ml of deionized water, and then photo-crosslinking the solution by using UV rays. The size of it was 1 cm × 1 cm, and the thickness of it was 1 mm. In addition, the mixture may be dissolved in 100 ml of saline solution instead of deionized water. In this case, the gel can be used in the living body without wasting time and labor required to substitute water, which is impregnated into the gel, with saline solution.

The saline solution-containing polyacrylamide gel (Saline poly-acrylamide gel) was obtained by adding 1 mg of a photo-crosslinking agent to 300 mg of polyacrylamide, dissolving the mixture in 100 ml of saline solution, and then photo-crosslinking the solution by using UV rays. The size of it was 1 cm × 1 cm, and the thickness of it was 1 mm.

The saline solution-containing rotaxane gel (Saline-rotaxane gel) was obtained by adding 1 mg of a photo-crosslinking agent to 300 mg of rotaxane gel, dissolving the mixture in 100 ml of saline solution, and then photo-crosslinking the solution by using UV rays. The size of it was 1 cm × 1 cm, and the thickness of it was 1 mm.

As shown in FIG. 4, it can be seen that the electrical capacitance of the composition according to the embodiment of the present invention is higher than those of the gels of the related art.

In addition, when an electrical signal is detected by capacitive coupling, the strength thereof is proportional to the surface area of the electrode. When an electrical signal is detected by capacitive coupling by using an electrode which is formed from the composition according to the present invention, the composition according to the present invention is far more flexible than a metal electrode of the related art, and the electrode can be closely attached onto a biological tissue and thus has a large substantial contact area. Therefore, the detection sensitivity at a substantial capacity for obtaining an electrical signal is extremely higher than that of a metal electrode of the related art, and thus high detectability can be obtained even in a small electrode.

In addition, the composition or the conductive material according to the present invention includes the carbon nanomaterial, and the carbon nanomaterial, particularly, the carbon nanotubes have a high specific surface area. Therefore, even from this point of view, high signal detectability can be obtained. In addition, the conductivity of an electrode, which is manufactured from the composition or the conductive material according to the present invention, is lower than that of an Au electrode. However, when a signal is detected by capacitance, it is important that a effective surface area, rather than the conductivity, is large.

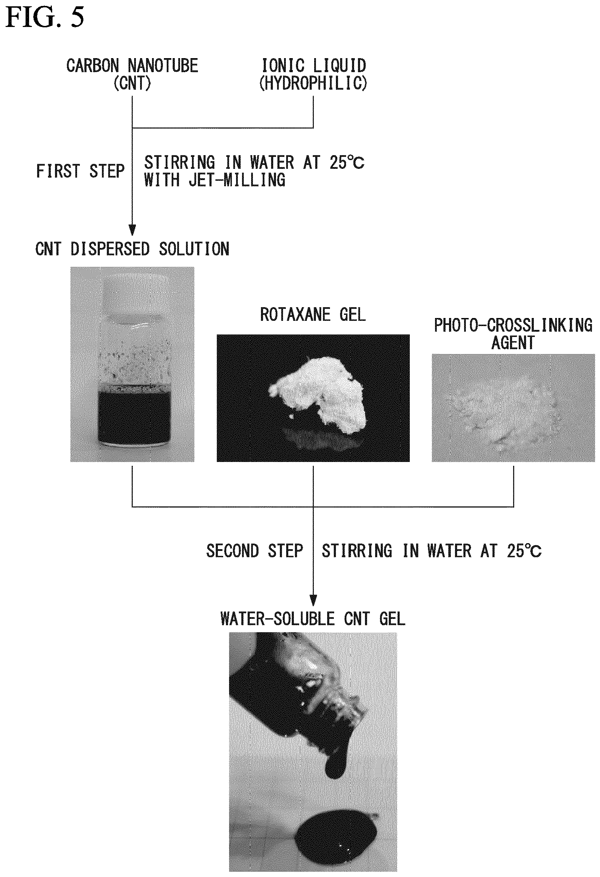

Hereinafter, the method of manufacturing a conductive material according to the embodiment of the present invention will be described with reference to FIG. 5 by using an example in which carbon nanotubes are used as the carbon nanomaterial, N, N-diethyl-N-methyl-N-(2-methoxyethyl)ammonium tetrafluoroborate (DEMEBF₄) is used as the ionic liquid, and polyrotaxane is used as the water-soluble polymer.

### (1) First Step

First, carbon nanotubes, DEMEBF₄, and water are mixed with each other and stirred to prepare a first dispersion system in which the carbon nanomaterial covered with the molecules constituting the ionic liquid is dispersed.

In the rinsing step, DEMEBF₄ which is not bonded to the carbon nanotubes may be removed by rinsing the first dispersion system by using, for example, saline solution, ethanol, or liquid which does not destroy gel.

In this dispersion system, the carbon nanotubes covered with the molecules constituting the ionic liquid are dispersed in water. Depending on the carbon nanotubes and the amount of the ionic liquid, the dispersion system may further include: carbon nanotubes (including bundles of carbon nanotubes) which are not sufficiently covered or are not covered at all with the molecules constituting the ionic liquid; and the molecules constituting the molecules constituting.

In this step, it is preferable that the carbon nanotubes be pulverized by applying a shearing force thereto by using a jet mill or the like. Due to this step, the degree of bundling (aggregation) is decreased, and the bundles which are formed due to the Van der Waals force can be separated into individual carbon nanotubes.

FIG. 7 shows the results of investigating the dispersibility of carbon nanotubes. (A) shows a state after 30 mg of carbon nanotubes were put into deionized water at 25°C and were stirred with a magnetic stirrer at a rotating speed of 700 rpm or higher for 1 week; (B) shows a state after 30 mg of carbon nanotubes and 60 mg of DEMEBF₄ were put into deionized water at 25°C and were stirred for 1 week in the same manner; (C) shows a state after 30 mg of carbon nanotubes were put into deionized water at 25°C, were stirred for 1 week in the same manner, and were processed with a high-pressure jet-milling homogenizer (60 MPa; Nano-Jet Pal, JN10, Jokoh); (D) shows a state after 30 mg of carbon nanotubes and 60 mg of DEMEBF₄ were put into deionized water at 25°C, were stirred for 1 week in the same manner, and were processed with a high-pressure jet-milling homogenizer; and (E) shows a state after 30 mg of carbon nanotubes, 60 mg of DEMEBF₄, and micro fibrillated cellulose (100 mg of an aqueous solution containing 10% cellulose, "Celish (trade name)", manufactured by Daicel Chemical Industries, Ltd.) were put into deionized water at 25°C and were stirred for 1 week in the same manner to obtain a paste, and the paste was processed with a high-pressure jet-milling homogenizer. The images were taken 1 week after the completion of stirring. "Celish (trade name)" is cellulose nanofiber which is obtained by microfibrillating a raw material of highly refined pure plant fiber by using a special processing method. Due to this processing, the raw material fiber is split into several tens of thousand pieces and is pulverized such that the thickness of the fiber is 0.1 µm to 0.01 µm.

It can be seen that (D) and (E) show high dispersibility of the carbon nanotubes in water. It can be seen that, in order to obtain high dispersibility, it is preferable that bundles of carbon nanotubes be pulverized by applying a shearing force thereto.

### (2) Second Step

Next, the first dispersion system, polyrotaxane ("photo-crosslinkable gel", manufactured by Advanced Softmaterials Inc.), and water are mixed with each other and are stirred to prepare a second dispersion system in which the carbon nanomaterial covered with the molecules constituting the ionic liquid and the water-soluble polymer are dispersed.

In the rinsing step, DEMEBF₄ which is not bonded to the carbon nanotubes may be removed by rinsing the second dispersion system by using, for example, saline solution, ethanol, or liquid which does not destroy gel.

When the obtained composition is crosslinked as shown in FIG. 5, a crosslinking agent is further mixed. As a result, the obtained second dispersion system is a gel material as shown in FIG. 5.

### (3) Crosslinking Step

Next, polyrotaxane is crosslinked, and the carbon nanotubes, which is covered with the molecules constituting DEMEBF₄, are dispersed in a polyrotaxane medium to obtain a composition (conductive material) in which polyrotaxane is crosslinked.

In the rinsing step, DEMEBF₄ which is not bonded to the carbon nanotubes may be removed by rinsing the obtained composition (conductive material) by using, for example, saline solution, ethanol, or liquid which does not destroy gel.

Through the above-described steps, the composition (conductive material) according to the embodiment of the present invention can be obtained.

Next, an example of a step of using the above-described second dispersion system to form a sheet which is formed of the composition (conductive material) according to the embodiment of the present invention or to form a line having a fine width which is formed of the composition (conductive material) according to the embodiment of the present invention will be described.

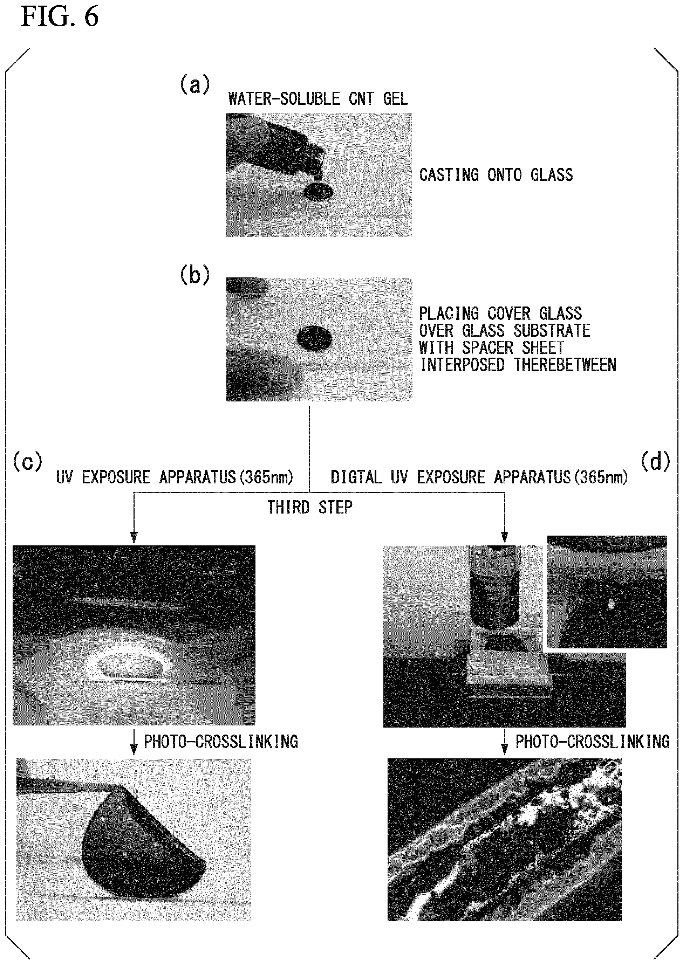

As shown in FIG. 6(a), the second dispersion system is cast onto a glass substrate. Next, as shown in FIG. 6(b), a cover glass is placed over the glass substrate with a spacer sheet having a desired thickness (in an example of the drawing, 50 µm) interposed therebetween.

Next, in order to form a sheet, the glass substrate is exposed to, for example, ultraviolet rays (365 nm) by using an ultraviolet exposure apparatus as shown in FIG. 6(c). As a result, a sheet having a thickness of 50 µm can be obtained. In addition, in order to form a line having a fine width, the glass substrate is exposed to, for example, ultraviolet rays (365 nm) by using a digital ultraviolet exposure apparatus as shown in FIG. 6(d). As a result, a line having a width of 50 µm can be formed.

### Industrial Applicability

According to the present invention, a carbon nanomaterial, a composition, and a conductive material which have sufficient conductivity and flexibility can be provided. Further, a carbon nanomaterial, a composition, and a conductive material which have biocompatibility can be provided. Accordingly, the present invention is industrially extremely useful.

## Claims

1. A carbon nanomaterial covered with polymer comprising:
a layer of molecules constituting a hydrophilic ionic liquid, the layer covering a surface of the carbon nanomaterial,
wherein the layer is covered with water-soluble polymer in the form of a layer, the water-soluble polymer being capable of being dissolved or dispersed in water.

2. The carbon nanomaterial covered with polymer according to claim 1,
wherein the water-soluble polymer is polyrotaxane.

3. The carbon nanomaterial covered with polymer according to claim 1 or 2,
wherein the water-soluble polymer is crosslinked.

4. A composition comprising a carbon nanomaterial covered with polymer according to any one of claims 1 to 3 dispersed in a water-soluble polymer medium.

5. The composition according to claim 4,
wherein the composition is gel or liquid.

6. The composition according to claim 4 or 5,
wherein the carbon nanomaterial is carbon nanotubes.

7. A biocompatible composition consisting of
the composition according to any one of claims 4 to 6.

8. A conductive material comprising a carbon nanomaterial covered with polymer according to any one of claims 1 to 3 dispersed in a water-soluble polymer medium, the water-soluble polymer being crosslinked.

9. A biocompatible conductive material comprising a carbon nanomaterial covered with polymer according to any one of claims 1 to 3 dispersed in a water-soluble polymer medium, the water-soluble polymer being crosslinked.

10. A method of manufacturing a conductive material comprising:
a first step of mixing a hydrophilic ionic liquid, a carbon nanomaterial, and water with each other to prepare a carbon nanomaterial whose surface is covered with a layer of molecules constituting an ionic liquid;
a second step of mixing the carbon nanomaterial obtained in the first step whose surface is covered with the molecules constituting the ionic liquid, a water-soluble polymer, and water with each other and dispersing the carbon nanomaterial, in which the layer of the molecules constituting the ionic liquid is covered with the water-soluble polymer in the form of a layer, in a water-soluble polymer medium.

11. The method of manufacturing a conductive material according to claim 10,
wherein in the first step, the carbon nanomaterial is pulverized by applying a shearing force to the carbon nanomaterial.

12. The method of manufacturing a conductive material according to claim 10 or 11, further comprising:
a third step of crosslinking the water-soluble polymer by exposing the water-soluble polymer medium to ultraviolet rays.

13. The method of manufacturing a conductive material according to any one of claims 10 to 12,
wherein the water-soluble polymer is polyrotaxane.

14. The method of manufacturing a conductive material according to any one of claims 10 to 13,
further comprising:
a rinsing step of removing the molecules constituting the ionic liquid which are not bonded to the carbon nanomaterial.

15. A use of a carbon nanomaterial covered with polymer as a biocompatible material, the carbon nanomaterial covered with the polymer comprising:
a layer of molecules constituting a hydrophilic ionic liquid, the layer covering a surface of the carbon nanomaterial,
wherein the layer is covered with polyrotaxane in the form of a layer.

16. A use of a composition as a biocompatible material, the composition comprising:
a carbon nanomaterial whose surface is covered with a layer of molecules constituting a hydrophilic ionic liquid, the carbon nanomaterial being dispersed in a water-soluble polymer medium,
wherein the layer of the molecules constituting the ionic liquid is covered with a water-soluble polymer in the form of a layer, and
the water-soluble polymer is crosslinked.

## Patentansprüche

1. Mit einem Polymer bedecktes Kohlenstoff-Nanomaterial umfassend:
eine Schicht von Molekülen, welche eine hydrophile ionische Flüssigkeit darstellen, wobei die Schicht eine Oberfläche des Kohlenstoff-Nanomaterials bedeckt,
wobei die Schicht mit einem wasserlöslichen Polymer in Form einer Schicht bedeckt ist, und wobei das wasserlösliche Polymer in Wasser gelöst oder dispergiert werden kann.

2. Mit einem Polymer bedecktes Kohlenstoff-Nanomaterial gemäß Anspruch 1, worin das wasserlösliche Polymer Polyrotaxan ist.

3. Mit einem Polymer bedecktes Kohlenstoff-Nanomaterial nach Anspruch 1 oder 2, worin das wasserlösliche Polymer quervernetzt ist.

4. Zusammensetzung umfassend ein mit einem Polymer bedecktes Kohlenstoff-Nanomaterial gemäß einem der Ansprüche 1-3, dispergiert in einem wasserlöslichen Polymermedium.

5. Zusammensetzung nach Anspruch 4, worin die Zusammensetzung ein Gel oder eine Flüssigkeit ist.

6. Zusammensetzung gemäß Anspruch 4 oder 5, worin das Kohlenstoff-Nanomaterial Kohlenstoff-Nanotubes sind.

7. Biokompatible Zusammensetzung bestehend aus einer Zusammensetzung gemäß einem der Ansprüche 4 bis 6.

8. Leitendes Material umfassend ein mit einem Polymer bedecktes Kohlenstoff-Nanomaterial gemäß einem der Ansprüche 1 bis 3, dispergiert in einem wasserlöslichen Polymermedium, wobei das wasserlösliche Polymer quervernetzt ist.

9. Biokompatibles leitendes Material umfassend ein mit einem Polymer bedecktes Kohlenstoff-Nanomaterial gemäß einem der Ansprüche 1-3, dispergiert in einem wasserlöslichen Polymer-Medium, wobei das wasserlösliche Polymer quervernetzt ist.

10. Verfahren zur Herstellung eines leitfähigen Materials umfassend:
- einen ersten Schritt des miteinander Mischens einer hydrophilen ionischen Flüssigkeit, eines Kohlenstoff-Nanomaterials und Wasser zur Erzeugung eines Kohlenstoff-Nanomaterials, dessen Oberfläche mit einer Schicht von Molekülen bedeckt ist, welche eine ionische Flüssigkeit darstellt;
- einen zweiten Schritt des miteinander Mischens des in Schritt 1 erhaltenen Kohlenstoff-Nanomaterials, dessen Oberfläche mit Molekülen, welche eine ionische Flüssigkeit darstellen, bedeckt ist, eines wasserlöslichen Polymers und Wasser, und Dispergierens des Kohlenstoff-Nanomaterials, in welchem die Schicht der Moleküle, die eine ionische Flüssigkeit darstellen, mit dem wasserlöslichen Polymer in Form einer Schicht bedeckt ist, in einem wasserlöslichen Polymermedium.

11. Verfahren zur Herstellung eines leitfähigen Materials gemäß Anspruch 10,
worin in dem ersten Schritt das Kohlenstoff-Nanomaterial durch Anlegen einer Scherkraft an das Kohlenstoff-Nanomaterial pulverisiert wird.

12. Verfahren zur Herstellung eines leitfähigen Materials gemäß einem der Ansprüche 10 oder 11, weiter umfassend einen dritten Schritt des Quervernetzens des wasserlöslichen Polymers durch Aussetzen des wasserlöslichen Polymermediums einer ultravioletten Strahlung.

13. Verfahren zur Herstellung eines leitfähigen Materials gemäß einem der Ansprüche 10 bis 12, worin das wasserlösliche Polymer Polyrotaxan ist.

14. Verfahren zur Herstellung eines leitfähigen Materials gemäß einem der Ansprüche 10 bis 13, weiter umfassend:
einen Spülschritt zur Entfernung von Molekülen, die eine ionische Flüssigkeit darstellen, die nicht an das Kohlenstoff-Nanomaterial gebunden sind.

15. Verwendung eines mit einem Polymer bedeckten Kohlenstoff-Nanomaterials als ein biokompatibles Material, wobei das Kohlenstoff-Nanomaterial mit einem Polymer bedeckt ist, umfassend:
eine Schicht von Molekülen, welche eine hydrophile ionische Flüssigkeit darstellen, wobei die Schicht eine Oberfläche des Kohlenstoff-Nanomaterials bedeckt,
worin die Schicht mit Polyrotaxan in Form einer Schicht bedeckt ist.

16. Verwendung einer Zusammensetzung als ein biokompatibles Material, wobei die Zusammensetzung umfasst:
ein Kohlenstoff-Nanomaterial, dessen Oberfläche mit einer Schicht von Molekülen, welche eine hydrophile ionische Flüssigkeit darstellen, bedeckt ist, wobei das Kohlenstoff-Nanomaterial in einem wasserlöslichen Polymermedium dispergiert ist,
worin die Schicht der Moleküle, welche eine ionische Flüssigkeit darstellen, mit einem wasserlöslichen Polymer in Form einer Schicht bedeckt wird, und
das wasserlösliche Polymer quervernetzt ist.

## Revendications

1. Nanomatériau de carbone recouvert d'un polymère comprenant :
une couche de molécules constituant un liquide ionique hydrophile, la couche recouvrant une surface du nanomatériau de carbone,
dans lequel la couche est recouverte d'un polymère soluble dans l'eau sous la forme d'une couche, le polymère soluble dans l'eau étant capable d'être dissous ou dispersé dans l'eau.

2. Nanomatériau de carbone recouvert d'un polymère selon la revendication 1,
dans lequel le polymère soluble dans l'eau est le polyrotaxane.

3. Nanomatériau de carbone recouvert d'un polymère selon la revendication 1 ou 2, dans lequel le polymère soluble dans l'eau est réticulé.

4. Composition comprenant un nanomatériau de carbone recouvert d'un polymère selon l'une quelconque des revendications 1 à 3 dispersé dans un milieu de polymère soluble dans l'eau.

5. Composition selon la revendication 4,
dans laquelle la composition est un gel ou un liquide.

6. Composition selon la revendication 4 ou 5,
dans laquelle le nanomatériau de carbone est des nanotubes de carbone.

7. Composition biocompatible consistant en
la composition selon l'une quelconque des revendications 4 à 6.

8. Matériau conducteur comprenant un nanomatériau de carbone recouvert d'un polymère selon l'une quelconque des revendications 1 à 3 dispersé dans un milieu de polymère soluble dans l'eau, le polymère soluble dans l'eau étant réticulé.

9. Matériau conducteur biocompatible comprenant un nanomatériau de carbone recouvert d'un polymère selon l'une quelconque des revendications 1 à 3 dispersé dans un milieu de polymère soluble dans l'eau, le polymère soluble dans l'eau étant réticulé.

10. Procédé de fabrication d'un matériau conducteur comprenant :
une première étape d'un mélange d'un liquide ionique hydrophile, d'un nanomatériau de carbone et de l'eau les uns avec les autres pour préparer un nanomatériau de carbone dont la surface est recouverte d'une couche de molécules constituant un liquide ionique ;
une deuxième étape de mélange du nanomatériau de carbone obtenu dans la première étape dont la surface est recouverte des molécules constituant le liquide ionique, un polymère soluble dans l'eau, et de l'eau les uns avec les autres et dispersion du nanomatériau de carbone, dans lequel la couche des molécules constituant le liquide ionique est recouverte du polymère soluble dans l'eau sous la forme d'une couche, dans un milieu de polymère soluble dans l'eau.

11. Procédé de fabrication d'un matériau conducteur selon la revendication 10,
dans lequel, dans la première étape, le nanomatériau de carbone est pulvérisé par application d'une force de cisaillement au nanomatériau de carbone.

12. Procédé de fabrication d'un matériau conducteur selon la revendication 10 ou 11, comprenant en outre :
une troisième étape de réticulation du polymère soluble dans l'eau par exposition du milieu de polymère soluble dans l'eau à des rayons ultraviolets.

13. Procédé de fabrication d'un matériau conducteur selon l'une quelconque des revendications 10 à 12,
dans lequel le polymère soluble dans l'eau est le polyrotaxane.

14. Procédé de fabrication d'un matériau conducteur selon l'une quelconque des revendications 10 à 13,
comprenant en outre :
une étape de rinçage consistant à éliminer les molécules constituant le liquide ionique qui ne sont pas liées au nanomatériau de carbone.

15. Utilisation d'un nanomatériau de carbone recouvert d'un polymère en tant que matériau biocompatible, le nanomatériau de carbone recouvert du polymère comprenant :
une couche de molécules constituant un liquide ionique hydrophile, la couche recouvrant une surface du nanomatériau de carbone,
dans lequel la couche est recouverte de polyrotaxane sous la forme d'une couche.

16. Utilisation d'une composition en tant que matériau biocompatible, la composition comprenant :
un nanomatériau de carbone dont la surface est recouverte d'une couche de molécules constituant un liquide ionique hydrophile, le nanomatériau de carbone étant dispersé dans un milieu de polymère soluble dans l'eau,
dans lequel la couche des molécules constituant le liquide ionique est recouverte d'un polymère soluble dans l'eau sous la forme d'une couche, et
le polymère soluble dans l'eau est réticulé.
